# EUROPEAN PATENT APPLICATION

(11) **EP 1 671 944 A1**
(43) Date of publication of application: **21.06.2006**
(21) Application number: 05021175.4
(22) Date of filing: 28.09.2005
(51) Int. Cl.: C07C 253/30, C07C 255/37

(54) **Method of preparing 1-(cyano(p-methoxyphenyl)ethyl)cyclohexanol**

(30) Priority: 15.12.2004 KR 2004106434
(71) Applicant: SK Corporation, Seoul 110-110 (KR)
(72) Inventor: Kwak, Byong Sung, Yuseong-gu Daejeon 305-761 (KR); Chung, Ki Nam, Yuseong-gu Daejeon 305-761 (KR); Choi, Jun Tae, Yuseong-gu Daejeon 305-729 (KR); Lim, Jong Ho, Seo-gu Daejeon 302-122 (KR)
(74) Representative: Fiesser, Gerold Michael

(57) **Abstract**

Disclosed herein is a method of preparing 1-[cyano(p-methoxyphenyl)ethyl]cyclohexanol. Particularly, the method of this invention includes reacting p-methoxyphenylacetonitrile with cyclohexanone in the presence of a solvent mixture including an aqueous solution of basic material and aqueous alcohol, to prepare 1-[cyano(p-methoxyphenyl)ethyl]cyclohexanol represented by Formula 1 below. According to this method, 1-[cyano(p-methoxyphenyl)ethyl]cyclohexanol, which is an important intermediate for use in the preparation of venlafaxine, exhibiting excellent efficacy as an antidepressant, may be very purely and economically prepared, and as well, may be industrially mass produced.

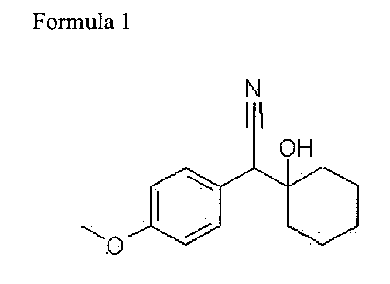

## Description

### BACKGROUND OF THE INVENTION

### 1. Field of the Invention

The present invention relates, generally, to a method of preparing 1-[cyano(p-methoxyphenyl)ethyl]cyclohexanol, and, more particularly, to a method of preparing 1-[cyano(p-methoxyphenyl)ethyl]cyclohexanol, including reacting p-methoxyphenylacetonitrile with cyclohexanone in the presence of a solvent mixture comprising an aqueous solution of basic material and aqueous alcohol, to very purely and economically mass produce 1-[cyano(p-methoxyphenyl)ethyl]cyclohexanol, which is an important intermediate for use in the preparation of venlafaxine serving as an antidepressant.

### 2. Description of the Related Art

In general, venlafaxine is known to be an antidepressant exhibiting excellent efficacies as a serotonin and norepinephrine reuptake inhibitor, despite its simple molecular structure. Venlafaxine may be prepared via a variety of preparation paths, and, in particular, may be easily prepared using 1-[cyano(p-methoxyphenyl)ethyl]cyclohexanol. As such, 1-[cyano(p-methoxyphenyl)ethyl]cyclohexanol may result from a single reaction of p-methoxyphenylacetonitrile and cyclohexanone. Many techniques for preparing 1-[cyano(p-methoxyphenyl)ethyl]cyclohexanol using p-methoxyphenylacetonitrile and cyclohexanone are known.

U.S. Patent No. 4,535,186 discloses a method of preparing 1-[cyano(p-methoxyphenyl)ethyl]cyclohexanol, comprising reacting p-methoxyphenylacetonitrile with n-butyllithium to prepare p-methoxyphenylacetonitrile anions, which are then reacted with cyclohexanone, to finally obtain 1-[cyano(p-methoxyphenyl)ethyl]cyclohexanol. However, n-butyllithium is very sensitive to water and is thus easily decomposed, and is also very dangerous due to its flammability. Hence, this material should be very carefully handled when used industrially. In addition, since the reaction temperature should be maintained at -50°C or less, preparation equipment is complicated and the preparation cost increases. Consequently, the above method is difficult to use industrially.

U.S. Patent No. 5,043,466 discloses a method of preparing 1-[cyano(p-methoxyphenyl)ethyl]cyclohexanol, comprising reacting p-methoxyphenylacetonitrile with lithium diisopropylamine to produce p-methoxyphenylacetonitrile anions, which are then reacted with cyclohexanone, thus obtaining 1-[cyano(p-methoxyphenyl)ethyl]cyclohexanol. Although this method is advantageous because the reaction may be conducted at about 10°C, lithium diisopropylamine is expensive and is also easily decomposed due to its sensitivity to water. Consequently, this method is difficult to use industrially.

In preparation methods, which were mainly developed in India and China and are disclosed in EP No. 1,238,967 and WO 02/18325 and WO 03/080565, an inexpensive aqueous basic material such as sodium hydroxide and a small amount of phase transfer catalyst are used in an aqueous solution, thus solving problems of conventional methods. However, while the reaction is progressed, all reactants or large amounts thereof may be solidified in the reactor. Thus, the above methods further require a process of grinding the solidified reactants, and therefore, are unsuitable for mass production and are difficult to use industrially.

In addition, although a preparation method disclosed in WO 03/000652 is advantageous because a safe inorganic metal amine base such as 1,8-diazabicyclo[5,4,0]undec-7-ene (DBU) is used and other solvents in addition to the raw material are not used, it suffers because the inorganic metal amine base used is expensive. Hence, this method is unsuitable for industrial use.

### SUMMARY OF THE INVENTION

Leading to the present invention, extensive and intensive research into methods of preparing 1-[cyano(p-methoxyphenyl)ethyl]cyclohexanol, carried out by the present inventors aiming to avoid the problems encountered in the prior art, resulted in the finding that a phase transfer catalyst need not be used, unlike conventional methods, and thus, a solidification phenomenon does not occur, and also, p-methoxyphenylacetonitrile may be reacted with cyclohexanone in the presence of aqueous alcohol and an inexpensive basic material which is less dangerous and easily handleable, to very purely, economically and safely mass prepare 1-[cyano(p-methoxyphenyl)ethyl]cyclohexanol, which is an important intermediate for use in the preparation of venlafaxine serving as an antidepressant.

Accordingly, an object of the present invention is to provide a method of preparing highly pure 1-[cyano(p-methoxyphenyl)ethyl]cyclohexanol, which is economical and is suitable for industrial application.

In order to achieve the above object, the present invention provides a method of preparing 1-[cyano(p-methoxyphenyl)ethyl]cyclohexanol, including reacting p-methoxyphenylacetonitrile with cyclohexanone in the presence of a solvent mixture comprising an aqueous solution of basic material and aqueous alcohol, to obtain solid particles, which are then filtered, to prepare 1-[cyano(p-methoxyphenyl)ethyl]cyclohexanol represented by Formula 1 below:

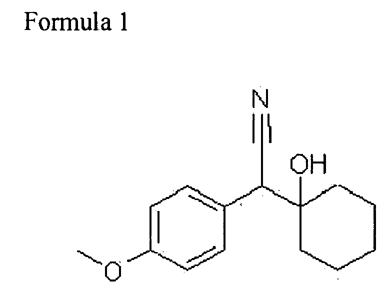

### DESCRIPTION OF THE PREFERRED EMBODIMENTS

Hereinafter, a detailed description will be given of the present invention.

In the present invention, a method of preparing 1-[cyano(p-methoxyphenyl)ethyl]cyclohexanol comprises reacting p-methoxyphenylacetonitrile with cyclohexanone in the presence of a solvent mixture including an aqueous solution of basic material and aqueous alcohol.

According to the present invention, p- methoxyphenylacetonitrile and cyclohexanone are added to the solvent mixture including an aqueous solution of basic material obtained by dissolving a basic material in water and aqueous alcohol, and are then allowed to react with stirring, so that an organic material layer is efficiently dispersed in the aqueous solution. Subsequently, the resultant small solid particles are filtered, whereby 1-[cyano(p-methoxyphenyl)ethyl]cyclohexanol as a solid product may be obtained at a high yield. During the reaction, a phenomenon where all reactants or large amounts thereof are solidified in a reactor has not been observed.

The basic material is lithium hydroxide, sodium hydroxide, potassium hydroxide, calcium hydroxide, sodium hydrogen carbonate, potassium hydrogen carbonate, sodium carbonate, or potassium carbonate. Preferably, sodium hydroxide or potassium hydroxide may be used. As such, the aqueous solution of basic material includes 1-45%, preferably, 5-30%, and more preferably, 10-20% basic material. If the concentration of basic material in an aqueous solution is less than 1%, water is used in too large an amount, so that the preparation method is difficult to use industrially. On the other hand, if the above concentration exceeds 45%, side reactions occur, thus the amount of impurities excessively increases. The basic material is added in an amount of 0.3-1.0 mol, based on the p-methoxyphenylacetonitrile. The use of the basic material less than 0.3 mol results in a slow reaction rate, whereas the use of the basic material exceeding 1.0 mol results in a fast reaction rate. However, side reactions may occur, thus increasing the amount of impurities.

The aqueous alcohol used in the present invention is selected from the group consisting of methanol, ethanol, isopropanol, n-propanol, ethyleneglycol, glycerol, propanediol, butanediol, butanetriol, ethyleneglycol monomethylether, ethyleneglycol monoethylether, ethyleneglycol monobutylether, polyethyleneglycol, polyethyleneglycol monomethylether, polyethyleneglycol monobutylether, polypropyleneglycol, polypropyleneglycol monomethylether, monosaccharide carbohydrates such as glucose, fructose, mannose, galactose, or ribose, and mixtures thereof.

The concentration of aqueous alcohol, which is mixed with water, is preferably 1-30%, and more preferably, 5-20%. If the concentration of aqueous alcohol is less than 1%, a reaction rate is too slow. Meanwhile, if the above concentration exceeds 30%, a reaction rate is fast, but impurities are produced in a larger amount by the side reactions. Moreover, the produced 1-[cyano(p-methoxyphenyl)ethyl]cyclohexanol may be decomposed again into p-methoxyphenylacetonitrile and cyclohexanone, therefore reducing the yield.

In addition, the cyclohexanone is preferably added in an amount of 1-2 mol, based on the p-methoxyphenylacetonitrile. If the amount of cyclohexanone is less than 1 mol, a conversion rate of p-methoxyphenylacetonitrile decreases. On the other hand, if the above amount exceeds 2 mol, the amount of unreacted cyclohexanone increases, thus negating economic benefits.

The reaction of the present invention is preferably conducted at 0-40°C. A reaction temperature lower than 0°C results in a slow reaction rate, whereas a temperature exceeding 40°C results in the occurrence of side reactions, thus increasing the amount of impurities. Further, 1-[cyano(p-methoxyphenyl)ethyl]cyclohexanol, which is a final product, may be decomposed again into p-methoxyphenylacetonitrile and cyclohexanone, thus the yield is reduced.

As mentioned above, the present invention is directed to a method of preparing 1-[cyano(p-methoxyphenyl)ethyl]cyclohexanol, by reacting p-methoxyphenylacetonitrile and cyclohexanone with a basic material in a solvent mixture including aqueous alcohol and water.

The method of the present invention is advantageous from process and economical points of view because less dangerous and inexpensive basic material is used in the form of an aqueous solution, and a phase transfer catalyst is not used, unlike conventional methods, and thus, an additional treatment process due to the solidification of the reactants is not needed. That is, according to the method of the present invention, 1-[cyano(p-methoxyphenyl)ethyl]cyclohexanol, which is an important intermediate of venlafaxine serving as an antidepressant, may be economically prepared, and industrially mass produced, as well.

A better understanding of the present invention may be obtained in light of the following examples which are set forth to illustrate, but are not to be construed to limit the present invention.

### Example 1

Into a 250 ml reactor, 30 ml of water and 2.95 g of sodium hydroxide were loaded, to prepare an aqueous solution of sodium hydroxide. Then, 3 ml of polyethyleneglycol monomethylether were added to the above aqueous solution, and the temperature of a solvent mixture was maintained at 20°C. Subsequently, 20 ml of p-methoxyphenylacetonitrile and 21 ml of cyclohexanone were added to the solvent mixture, and were then allowed to react with stirring for 8 hr, so that an organic layer was efficiently dispersed in the aqueous solution. As a result, a suspension having fine solids dispersed therein was produced. The reaction mixture was filtered using a filter, after which a solid layer was washed with 30 ml of water, to recover solid material. The recovered solid material was dissolved in 30 ml of dichloromethane, and then 60 ml of n-heptane were added. Most dichloromethane was removed through evaporation under reduced pressure, and the remaining material was filtered, thus yielding 31.85 g of 1-[cyano(p-methoxyphenyl)ethyl]cyclohexanol as white crystal. The solid product thus obtained was confirmed to have 99.7% chemical purity through liquid chromatography, and was also confirmed to be 1-[cyano(p-methoxyphenyl)ethyl]cyclohexanol through nuclear magnetic resonance analysis.

¹H NMR(200MHz, CDCl₃) 1.56 (m, 10H), 3.76 (s, 1H), 3.80 (s, 3H), 6.95 (s, 2H), 7.34 (s, 2H).

### Example 2

Into a 50 ml reactor, 6.6 ml of water and 0.7 g of potassium hydroxide were loaded, to prepare an aqueous solution of potassium hydroxide. Then, 0.7 ml of polyethyleneglycol monomethylether were added to the above aqueous solution, and the reaction temperature was maintained at 25°C. Subsequently, 2.5 ml of p-methoxyphenylacetonitrile and 2.7 ml of cyclohexanone were added, and were then allowed to react with stirring for 5 hr, so that an organic layer was efficiently dispersed in the aqueous solution. As a result, a suspension having fine solids dispersed therein was produced. The reaction mixture was filtered using a filter, after which a solid layer was washed with 30 ml of water, to recover solid material. The recovered solid material was dissolved in 1 ml of dichloromethane, and then 10 ml of n-heptane were added. Most dichloromethane was removed through evaporation under reduced pressure, and then the remaining material was filtered, thus yielding 1.7 g of 1-[cyano(p-methoxyphenyl)ethyl]cyclohexanol as white crystal. As in Example 1, the solid product thus obtained was confirmed to have 99.5% chemical purity through liquid chromatography, and was also confirmed to be 1-[cyano(p-methoxyphenyl)ethyl]cyclohexanol through nuclear magnetic resonance analysis.

### Example 3

Into a 50 ml reactor, 6.6 ml of water and 0.7 g of potassium hydroxide were loaded, to prepare an aqueous solution of potassium hydroxide. Then, 0.7 ml of polyethyleneglycol monomethylether were added to the above aqueous solution, and the reaction temperature was maintained at 5°C. Subsequently, 2.5 ml of p-methoxyphenylacetonitrile and 2.7 ml of cyclohexanone were added, and were then allowed to react with stirring for 5 hr, so that an organic layer was efficiently dispersed in the aqueous solution. As a result, a suspension having fine solids dispersed therein was produced. The reaction mixture was filtered using a filter, after which a solid layer was washed with 10 ml of water, to recover solid material. The recovered solid material was dissolved in 3 ml of dichloromethane, and then 10 ml of n-heptane were added. Most dichloromethane was removed through evaporation under reduced pressure, and then the remaining material was filtered, thus yielding 3.6 g of 1-[cyano(p-methoxyphenyl)ethyl]cyclohexanol as white crystal. As in Example 1, the product thus obtained was confirmed to have 99.5% chemical purity through liquid chromatography, and was also confirmed to be 1-[cyano(p-methoxyphenyl)ethyl]cyclohexanol through nuclear magnetic resonance analysis.

### Example 4

Into a 50 ml reactor, 7 ml of water and 0.7 g of sodium hydroxide were loaded, to prepare an aqueous solution of sodium hydroxide. Then, 1.0 ml of ethanol was added to the above aqueous solution, and the reaction temperature was maintained at 25°C. Subsequently, 2.5 ml of p-methoxyphenylacetonitrile and 2.7 ml of cyclohexanone were added, and were then allowed to react with stirring, so that an organic layer was efficiently dispersed in the aqueous solution. After 4 hr, 1.3 g of 1-[cyano(p-methoxyphenyl)ethyl]cyclohexanol were confirmed to be produced. As in Example 1, the product thus obtained was confirmed to have 85.0% chemical purity through liquid chromatography, and was also confirmed to be 1-[cyano(p-methoxyphenyl)ethyl]cyclohexanol through nuclear magnetic resonance analysis.

### Example 5

Into a 2 L reactor, 700 ml of water and 74 g of sodium hydroxide were loaded, to prepare an aqueous solution of sodium hydroxide. Then, 75 ml of polyethyleneglycol monomethylether were added to the above aqueous solution, and the reaction temperature was maintained at 20°C. Subsequently, 500 ml of p-methoxyphenylacetonitrile and 420 ml of cyclohexanone were added, and were then allowed to react with stirring for 7 hr, so that an organic layer was efficiently dispersed in the aqueous solution. As a result, a suspension having fine solids dispersed therein was produced. The reaction mixture was filtered using a filter, after which a solid layer was washed with 650 ml of water, to recover solid material. The recovered solid material was dissolved in 400 ml of dichloromethane, and then 1000 ml of n-heptane were added. Most dichloromethane was removed through evaporation under reduced pressure, and then the remaining material was filtered, thus yielding 788 g of 1-[cyano(p-methoxyphenyl)ethyl]cyclohexanol as white crystal. As in Example 1, the product thus obtained was confirmed to have 99.5% chemical purity through liquid chromatography, and was also confirmed to be 1-[cyano(p-methoxyphenyl)ethyl]cyclohexanol through nuclear magnetic resonance analysis.

As is apparent from Examples 1 to 5, highly pure 1-[cyano(p-methoxyphenyl)ethyl]cyclohexanol can be economically and simply prepared according to the present invention.

As described hereinbefore, the present invention provides a method of preparing 1-[cyano(p-methoxyphenyl)ethyl]cyclohexanol. According to the method of the present invention, when 1-[cyano(p-methoxyphenyl)ethyl]cyclohexanol is prepared, less dangerous and inexpensive basic material may be used in the form of an aqueous solution, and as well, a phase transfer catalyst need not be used, thus preventing the solidification of reactants. Hence, compared to conventional techniques, the method of the present invention is more preferable from process and economical points of view, and is thus suitable for mass production. In addition, the method of the present invention is industrially applied, whereby an important intermediate used in the industrial preparation of venlafaxine exhibiting excellent antidepression efficacies can be economically and easily prepared.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. A method of preparing 1-[cyano(p-methoxyphenyl)ethyl]cyclohexanol, comprising reacting p-methoxyphenylacetonitrile with cyclohexanone with stirring in the presence of a solvent mixture including an aqueous solution of basic material and aqueous alcohol, to obtain solid particles, which are then filtered, to produce 1-[cyano(p-methoxyphenyl)ethyl]cyclohexanol represented by Formula 1 below:

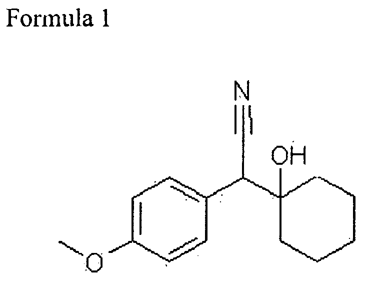

2. The method as set forth in claim 1, wherein the basic material is lithium hydroxide, sodium hydroxide, potassium hydroxide, calcium hydroxide, sodium hydrogen carbonate, potassium hydrogen carbonate, sodium carbonate, or potassium carbonate.

3. The method as set forth in claim 1, wherein the aqueous solution of basic material includes 1-45% basic material.

4. The method as set forth in claim 1, wherein the basic material is added in an amount of 0.3-1.0 mol, based on p-methoxyphenylacetonitrile.

5. The method as set forth in claim 1, wherein the aqueous alcohol is selected from the group consisting of methanol, ethanol, isopropanol, n-propanol, ethyleneglycol, glycerol, propanediol, butanediol, butanetriol, ethyleneglycol monomethylether, ethyleneglycol monoethylether, ethyleneglycol monobutylether, polyethyleneglycol, polyethyleneglycol monomethylether, polyethyleneglycol monobutylether, polypropyleneglycol, polypropyleneglycol monomethylether, glucose, fructose, mannose, galactose, ribose, and mixtures thereof.

6. The method as set forth in claim 1, wherein the solvent mixture includes 1-30% aqueous alcohol.

7. The method as set forth in claim 1, wherein the cylcohexanone is added in an amount of 1-2 mol, based on p-methoxyphenylacetonitrile.

8. The method as set forth in claim 1, wherein the reacting is performed at 0-40°C.
